# EUROPEAN PATENT SPECIFICATION

(11) **EP 2 617 708 B1**
(45) Date of publication and mention of the grant of the patent: **16.08.2017**
(21) Application number: 12151354.3
(22) Date of filing: 17.01.2012
(51) Int. Cl.: C07C 273/04

(54) **A method of forming urea by integration of an ammonia production process in a urea production process and a system therefor**
Verfahren zur Bildung von Harnstoff durch Integration eines Ammoniakherstellungsverfahrens in ein Harnstoffherstellungsverfahren sowie System dafür
Procédé de formation d'urée par intégration d'un procédé de production d'ammoniac dans un procédé de production d'urée et système associé

(43) Date of publication of application: 24.07.2013
(73) Proprietor: General Electric Technology GmbH, 5400 Baden (CH)
(72) Inventor: Ennenbach, Frank, 63303 Dreieich (DE); Koss, Ulrich, 8702 Zollikon (CH)
(74) Representative: General Electric Technology GmbH

(56) References cited:
- WO-A1-2008/135150
- GB-A- 1 359 715

## Description

### Field of the Invention

The present invention relates to a method of forming urea by integration of an ammonia production process in a urea production process, and to a system for said method. It also relates to a method of forming urea by integration of a part of an ammonia production process in a urea production process, and to a system for said method.

### Background of the Invention

In several combustion processes, and inwell-known ammonia production processes, a flue gas containing, inter alia, carbon dioxide is formed. This carbon dioxide component is often separated out or captured from the flue gas in one or more absorber systems using a solvent absorber. Typically, the spent carbon dioxide solvent absorber is then regenerated in one or more regeneration columns. In carbon dioxide capture systems of this kind, there is a need to reduce system energy consumption and capital expenditure ("capex") requirements. For such solvent-based carbon dioxide capture systems, much of the energy consumption and capex are due to the need for solvent absorber regeneration, i.e., carbon dioxide desorption, in regeneration columns. Regeneration columns have high energy consumption demands due to considerable heat requirements from a reboiler or other source for carbon dioxide desorption. Solvents typically used in solvent-based carbon dioxide capture systems include amines and ammonia. For example, in an ammonia production process a solvent-based carbon dioxide capture system known as the Chilled Ammonium Process (CAP) is normally used. In CAP, ammonia is used as the solvent. Other processes including a solvent-based carbon dioxide capture system use different kinds of amines or mixtures thereof.

WO 2006/022885 discloses ultra cleaning of combustion gas including removal of carbon dioxide in an absorbing subsystem with one or more carbon dioxide absorbing stages and a subsequent regeneration subsystem.

The process for urea production is well-known and extensively disclosed in the literature. Different variants of the urea production process exist, but a common feature is that ammonia and carbon dioxide are used as reactants under elevated pressure in a urea synthesis tower or reactor for the production of urea. According thereto, the following two equilibrium reactions take place:

2NH3 + CO₂ ⇄ NH₂COONH₄ (ammonium carbamate)

NH₂COONH₄ ⇄ NH₂CONH₂ (urea) + H₂O

After the urea synthesis reaction, urea, carbamate, water and excess ammonia are transported to a distillation reactor from which a solution or slurry of, inter alia, carbamate in equlibrum with ammonia, carbon dioxide and water is recycled back to the urea synthesis reactor for further reaction.

WO 2008/135150 discloses a method for purifying flue gases from incinerators and then producing urea. In this method, ammonia and flue gas containing carbon dioxide is added to a washing device in which carbon dioxide is absorbed. A solution containing (NH₄)₂CO₃ and (NH₄)₂SO₄ is emitted from the washing unit and transported to a stripper in which (NH₄)₂SO₄ is separated and (NH₄)₂CO₃ is subjected to an energy-consuming decomposition to carbon dioxide and ammonia. This carbon dioxide and ammonia is then used in a urea production process.

GB 1 359 715 discloses an integrated process for the production of ammonia and urea including process steps comprising the absorption of carbon dioxide from a concentrated ammonia solution with a view to yielding a concentrated ammonium carbamate solution to be fed to a urea reactor, wherein the carbon dioxide source is raw synthesis gas containing carbon dioxide and hydrogen.

Methods to reduce energy consumption and capex of solvent-based carbon dioxide capture systems have been developed, but the need for further cost reductions remain.

### Summary of the Invention

An object of the present invention is to provide a solvent-based carbon dioxide capture system and method with reduced energy consumption demands and capital cost requirements. This object is achieved by means of a system and method of forming urea by integration of an ammonia production process in a urea production process. As a result of this integration, ammonia and carbon dioxide obtained from synthetic gas ("syngas") is formed during steam reforming or gasification in connection with the ammonia production process in an ammonia production plant. Accordingly, in connection with an ammonia production process, a flue gas containing carbon dioxide and nitrogen from combustion is transported to a low pressure absorber and a hydrogen/carbon dioxide gas mixture from reforming steam is transported to a high pressure absorber. The high pressure absorber contains a solvent stream semi-rich with absorbed carbon dioxide. After contact with the hydrogen/carbon dioxide gas mixture, a solvent stream with ammonia and a rich or high content of carbon dioxide is emitted from the high pressure absorber and transported to a urea production plant. At the urea production plant, the transported carbon dioxide and ammonia are reacted for the production of urea. A recycle stream containing carbamate in a solution or slurry containing ammonia, carbon dioxide, and water is transported from the urea production process to a low pressure absorber. Likewise, a stream of a balance of ammonia is transported from the ammonia production unit to the low pressure absorber of the urea production plant. This alternative method is described in detail below.

According to one embodiment of the above described alternative method, a syngas stream containing hydrogen and carbon monooxide formed during the steam reforming reaction of the ammonia production process is transported to a carbon monooxide shift unit in which the carbon monooxide is converted into carbon dioxide to form the hydrogen/carbon dioxide stream.

According to one embodiment, a nitrogen containing stream is emitted from the urea production process low pressure absorber, and a hydrogen stream is emitted from the urea production process high pressure absorber. Both the nitrogen containing stream and the hydrogen stream are transported to the ammonia production unit and reacted for the production of ammonia.

According to one embodiment, an ammonia stream, optionally originating from the ammonia production unit, is added to the recycle stream used to generate a lean solvent stream containing ammonia and a relatively low content of carbon dioxide.

According to one embodiment, a slip stream diverted from the relatively rich solvent stream is transported to the low pressure absorber.

According to one embodiment, a partial stream of the relatively lean solvent stream is added to the high pressure absorber or to the semi rich solvent stream.

According to one embodiment, a partial stream of the semi rich solvent stream is transported to the urea production plant.

According to one embodiment, the pressure in the low pressure absorber is 0.08-0.15 MPa, preferably 0.095-0.11 MPa, and the pressure in the high pressure absorber is 0.3-4.0 MPa, preferably 0.095-0.11 MPa.

According to one embodiment, the concentration of ammonia in the relatively lean solvent stream, in the semi rich solvent stream and in the relatively rich solvent stream is 2- 12, preferably 6-9, moles per liter solution.

According to one embodiment, the concentration of carbon dioxide in the relatively lean solvent stream is 0.2-4.0, preferably 1.5-4.0, moles per liter solution, the concentration of carbon dioxide in the semi rich solvent stream is 1.0-5.5, preferably 2.5-4.5, moles per liter of solution, and the concentration of carbon dioxide in the relatively rich solvent stream is 1.0-10.0, preferably 3.0-6.0, moles per liter solution.

According to one embodiment, the ratio between the concentration of ammonia and the concentration of carbon dioxide in the lean, semi rich, and rich solvent streams, respectively, is 1:3, preferably 1.5:2.5.

A further object of the present invention is to provide systems in which the integrated method and the alternative integrated method are performed.

This further object is achieved by a system for the integrated method of forming urea by integration of an ammonia production process with that of a urea production process, wherein said system comprises an ammonia production plant, an absorber, and a urea synthesis reactor in a urea production plant. Said system includes a conduit for a first ammonia stream from the ammonia production plant separated into a conduit for a second ammonia stream fluidly connected to the absorber, or to a conduit for a lean solvent stream thus forming a mixed stream, and into a conduit for a third ammonia stream fluidly connected to the urea synthesis reactor. Likewise, a conduit for a flue gas stream containing carbon dioxide is fluidly connected between the ammonia production plant, or another flue gas source, and the absorber. A conduit for a carbon dioxide stream is fluidly connected between the ammonia production plant and the urea synthesis reactor. A conduit for a rich solvent stream is fluidly connected between the absorber and the urea synthesis reactor, and a conduit for a lean solvent stream is arranged between the urea production plant and the absorber.

According to one embodiment, this system comprises a conduit for a clean gas stream fluidly connected to the absorber.

This further object is achieved through an alternative integrated method with a system for forming urea by integration of a part of an ammonia production process with that of a urea production process. Such a system comprises a steam reformer or a gasification reactor of an ammonia production plant, a low pressure absorber, a high pressure absorber, and a urea production plant, wherein a conduit for a flue gas stream is fluidly connected between the steam reformer, or the gasification reactor, and the low pressure absorber. A conduit for a hydrogen/carbon dioxide stream is arranged between the steam reformer, the gasification reactor, or another flue gas source, and the high pressure absorber. A conduit for a semi rich solvent stream is fluidly connected between the low pressure absorber and the high pressure absorber. A conduit for a rich solvent stream is fluidly connected between the high pressure absorber and the urea production plant, and a conduit for a lean solvent stream is fluidly connected between the urea production plant and the low pressure absorber.

According to one embodiment of the alternative integrated method, the system comprises an ammonia production unit, a conduit for a hydrogen stream fluidly connected between the high pressure absorber and the ammonia production unit, and a conduit for a nitrogen containing stream fluidly connected between the low pressure absorber and the ammonia production unit. The system also comprises a conduit for a stream of the balance of ammonia fluidly connected between the ammonia production unit and the urea production plant.

According to one embodiment, the system comprises a carbon monooxide shift unit for the conversion of carbon monooxide into carbon dioxide, wherein a conduit for a syngas stream containing hydrogen and carbon monooxide is fluidly connected between the steam reformer, or the gasification reactor, and the carbon monooxide shift unit. Likewise, the conduit for the hydrogen/carbon dioxide stream is fluidly connected between the carbon monooxide shift unit and the high pressure absorber.

According to one embodiment, the system comprises a conduit for a partial stream of the semi rich solvent stream fluidly connected between the conduit for the semi rich solvent stream and the conduit for the rich solvent stream.

According to one embodiment, the system comprises a conduit for a recycle stream containing carbamate in a solution or in a slurry also containing ammonia, carbon dioxide and water, fluidly connected between the urea production plant and the conduit for the lean solvent stream. Likewise, a conduit for an ammonia stream is fluidly connected to the conduit for the recycle stream.

According to one embodiment, the system comprises a conduit for a partial stream of the lean solvent stream fluidly connected between the conduit for the lean solvent stream and the conduit for the semi rich solvent stream or the high pressure absorber.

### Brief description of the Drawings

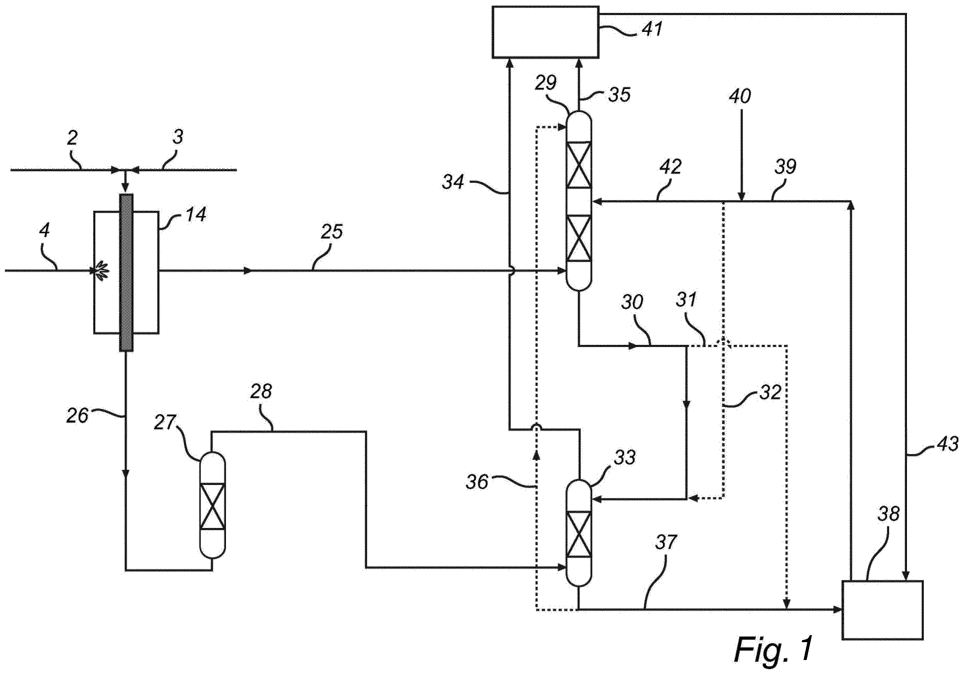

Referring now figure 1 which is an exemplary embodiment:
Fig. 1 is a schematic view of a system for the alternative integrated method of forming urea by integration of a part of an ammonia production process with a urea production process.

### Description of Preferred Embodiments

Carbon dioxide used as a reactant for a urea production process according to the present integrated method may originate from any combustion plant or apparatus, e.g., a conventional reformer. In one embodiment, the carbon dioxide originates from a steam reformer combustion apparatus part of an ammonia production process.

Syngas, also called synthetic gas or synthesis gas, is produced when hydrocarbons react with water in a reactor apparatus, e.g., a steam reformer. Syngas is a gas mixture containing varying amounts of carbon monooxide and hydrogen. A steam reformer used to produce syngas is a tubular reactor fired from the outside to providing sufficient reaction energy for a reforming reaction. For this purpose, a fuel is subjected to combustion in the fire box on the outer side of the steam reformer tubes. Inside the tubes of a steam reformer,the steam reforming reaction takes place in the presence of a catalyst to producesyngas. Carbon monooxide in the syngas is subjected to a carbon monooxide shift reaction with water to produce carbon dioxide. After such production, the carbon dioxide is then separated from the hydrogen. The separated hydrogen is then reacted with nitrogen in the air to produce ammonia. In addition to the use of hydrogen as a reactant for ammonia production and the use of carbon dioxide produced from the carbon monooxide shift reaction, useful flue gases are also formed from the combustion of the fuel for the steam reformer. These flue gases contain nitrogen, carbon dioxide and other contaminants, e.g., NOx. Normally, the carbon dioxide present in the flue gas in an ammonia production process can be absorbed in a CAP (Chilled Ammonia Process) absorber operating at atmospheric pressure and a low temperature. Before entering the CAP absorber, the flue gas is cooled to enhance absorption of the carbon dioxide by a carbon dioxide-lean ammoniated solution present in the absorber. After the carbon dioxide absorption step, the once carbon dioxide-lean ammoniated solution is a carbon dioxide rich solution that requires regeneration in a subsequent regeneration step. This process is disclosed more in detail in WO 2006/022885.

Another method for syngas production is gasification of a carbon or hydrocarbon with oxygen in a gasification reactor. Here, the reaction energy is provided within the gasification reactor itself. Syngas so produced can be treated as described above to separate the hydrogen from the carbon dioxide. However, in accordance with the present process there is a need for another flue gas source to produce additional carbon dioxide. Under such circumstances, the same integration scheme as applied for steam reformers can be similarly applied hereto.

Thus, syngas used in the present method is either formed in a steam reformation reaction or in a gasification reaction. In the steam reformation reaction, flue gas is produced by combustion during steam reforming. In the gasification reaction, the flue gas may originate from any source. The language "ammonia production process" and "ammonia production plant" used herein is intended to cover both of these reactions.

The integrated method and system will now be disclosed more in detail with reference to Fig. 1. In the embodiment, carbon dioxide produced in the ammonia production process is not per se integrated with the urea production process. Instead, a rich solvent stream from the absorption of the gas mixture of hydrogen and carbon dioxide (formed via a carbon monooxide shift reaction), originating from syngas produced in a steam reformer 14 or a gasification reactor during the ammonia production process, is used for the integration.

More precisely, hydrogen is typically produced during syngas production by steam reforming or gasification. Typically, physical absorption or adsorption processes are applied to separate the carbon dioxide from the hydrogen in the gas mixture of hydrogen and carbon dioxide. This occurs at elevated pressures, often much higher than those used during carbon dioxide absorption from flue gas in a high pressure absorber. After separation of carbon dioxide, the hydrogen is reacted with nitrogen in the air to form ammonia.

In the present alternative integrated method, both the flue gas stream and the stream of the gas mixture of hydrogen and carbon dioxide, originating from the syngas stream, are subjected to carbon dioxide absorption. If the chilled ammonia process (CAP) is applied to absorb carbon dioxide from the stream of the gas mixture of carbon dioxide and hydrogen, it is also possible to use the lean solvent stream containing carbamate from the urea process, if necessary enriched with additional ammonia. Then a low pressure absorber for the flue gas and a high pressure absorber for the gas mixture of carbon dioxide and hydrogen are needed. Said lean solvent may be used either in parallel to the low pressure absorber for flue gas or in sequence, using a semi rich solvent for the absorption of carbon dioxide in the high pressure absorber. Both the semi rich solvent and the rich solvent formed after high pressure absorption is fed back to the urea production plant 15a, preferably direct to the urea synthesis reactor 15 therein.

The advantages of this alternative integrated method are as follows. First, a combination of the two absorption processes (of carbon dioxide from flue gas and from syngas, respectively) using the same kind of solvent and same kind of principle (wet absorption) is obtained. Second, any need for desorption of carbon dioxide from the rich solvent is eliminated, since this rich solvent is used for the urea reaction. The rich solvent already contains ammonia and carbon dioxide as reactants for the urea process significantly reducingassociated energy and equipment costs. Third, no "foreign" chemical is added, such as an amine, as used in some carbon capture processes.

Further, potentially the carbon dioxide lean and nitrogen rich flue gas stream following absorption can be used as feedstock for ammonia synthesis to replace at least a part of the air used for ammonia synthesis. Such could allow for increased ammonia production, since less oxygen containing air is then fed to the synthesis. Any smaller amounts of ammonia slippage into either the hydrogen stream or the nitrogen rich stream from the absorbers should not poison the ammonia synthesis reaction. As an option, a slip stream from the rich solution stream may be transported to the low pressure absorber to minimize ammonia slippage into the nitrogen rich stream.

This embodiment for the formation of urea by integration of a part of an ammonia production process with the urea production process is schematically illustrated in Fig. 1 and will be disclosed in detail below with reference to Fig. 1. Although a steam reformer unit 14 is illustrated in Fig. 1, such could, as an alternative be a gasification reactor in which syngas is formed. In that case, the flue gas used in the alternative integrated method is provided from any other source (not shown in Fig. 1), such as a boiler, e.g. a power boiler or any boiler supplying heat to a urea production plant. Referring to Fig. 1 a steam reformer 14 is provided with a feedstock 2, e.g., natural gas, oil, or hydrocarbons, and with steam 3. The steam reformer 14 is fired with a fuel 4, e.g., natural gas. During the reaction between carbonhydrates and steam in the steam reformer 14 a syngas containing hydrogen and carbon monooxide is produced. A syngas stream 26 is transported to a carbon monooxide shift unit 27, in which the carbon monooxide is subjected to a shift into carbon dioxide. The hydrogen/ carbon dioxide stream 28 so obtained is transported directly to a high pressure absorber 33 having a pressure of 0.3-4 MPa, preferably 1-2.5 MPa. Further, a flue gas containing nitrogen, carbon dioxide, and other contaminants is emitted from the steam reformer 14, and transported as flue gas stream 25 to a low pressure absorber 29 having a pressure of 0.08-0.15 MPa, preferably 0.095-0.11 MPa.

In the high pressure absorber 33 carbon dioxide is absorbed and is then emitted therefrom in a rich solvent stream 37 containing carbon dioxide in a high concentration, water, and ammonia. This rich solvent stream 37 is then transported under a high pressure as a reactant to the urea synthesis reactor 38a (not shown) in a urea production plant 38. A hydrogen stream 34 is emitted from the high pressure absorber 33 for use as a reactant in an ammonia production unit 41. Optionally, a slip stream 36 of rich solvent stream 37 is transported to the top of the low pressure absorber 29, where it is used to catch excess ammonia from the upcoming treated flue gas stream 25 as necessary, before the cleaned flue gas, i.e., nitrogen containing stream 35, is transported to the ammonia production unit 41 for usage.

In the low pressure absorber 29 carbon dioxide contained in the flue gas stream 25 is absorbed by means of lean solvent stream 42. Such leaves low pressure absorber 29 as a semi rich solvent stream 30 containing absorbed carbon dioxide in a medium concentration, water, and ammonia. This semi rich solvent stream 30 is then transported to high pressure absorber 33. Optionally, a partial stream 31 of the semi rich solvent stream 30 is transported to the urea production plant 38. Before entering the low pressure absorber 29, flue gas stream 25 may be subjected to one or more conventional purification steps, and is also cooled to a temperature of between 0 and 25°C, preferably to a temperature between 10 and 20°C. Cleaned flue gas containing nitrogen at a high percentage, which is emitted from the low pressure absorber 29, can be transported as the nitrogen containing stream 35 to the ammonia production unit 41. Thus, the hydrogen in the hydrogen stream 34 and the nitrogen in the nitrogen containing stream 35 are transported as reactants to the ammonia production unit 41 in which the reaction between hydrogen and nitrogen takes place to produce ammonia. A recycle stream 39 containing a mixture of ammonium carbamate, ammonium carbonate and ammonium bicarbonate, either in solution or in a slurry, also containing unreacted ammonia, carbon dioxide, carbonic acid and water originating from the urea production plant 38, is optionally provided with additional ammonia via an ammonia stream 40 from the ammonia production unit 41 forming a lean solvent stream 42 in the form of a ammonia solution containing carbon dioxide in a low concentration, ammonia, ammonium carbamate, ammonium carbonate, ammonium bicarbonate and water, wherein this lean solvent stream 42 is transported to the low pressure absorber 29. Optionally, a partial stream 32 of the lean solution stream 42 may be transported directly to the high pressure absorber 33 or to the semi rich solvent stream 30. The balance of ammonia from the ammonia production unit 41 is transported in partial-stream 43 to the urea production plant 38.

The concentration of ammonia in the lean solvent stream 42, in the semi rich solvent stream 30 and in the rich solvent stream 37 is 2-12, preferably 6-10, moles per liter of solution. The concentration of carbon dioxide in the lean solvent stream 42 is 0.2-4.0, preferably 1.5-4, moles per liter solution. The concentration of carbon dioxide in the semi rich solvent stream 30 is 1.0-5.5, preferably 2.5-4.5, moles per liter solution. The concentration of carbon dioxide in the rich solvent stream 13 is 1.0-10.0, preferably 3.0-6.0, moles per liter solution. The ratio between the concentration of ammonia and the concentration of carbon dioxide in stream 37 is preferably 1 ;1.5 to 1:2.5 in order to satisfy the stochiometrics of the urea reaction.

To summarize, the present invention relates to a method of forming urea by integration of an ammonia production process in a urea production process, and to a system for said method. It also refers to an alternative method of forming urea by integration of a part of an ammonia production process in a urea production process, and to a system for said alternative method.

While the invention has been described with reference to a number of preferred embodiments, it will be understood by those skilled in the art that various changes may be made and equivalents may be substituted for elements thereof without departing from the scope of the present invention. In addition, many modifications may be made to adapt a particular situation or material to the teachings of the invention without departing from the essential scope thereof. Therefore, it is intended that the invention not be limited to the particular embodiments disclosed as the best mode contemplated for carrying out this invention, but that the invention will include all embodiments falling within the scope of the appended claims.

## Claims

1. A method of forming urea by integration of a part of an ammonia production process in a urea production process, comprising:
obtaining a hydrogen/carbon dioxide stream (28) from an ammonia production process, and a flue gas stream (25) containing carbon dioxide and nitrogen,
transporting flue gas stream (25) to a low pressure absorber (29),
transporting hydrogen/carbon dioxide stream (28) to a high pressure absorber (33) with a semi rich solvent stream (30) containing absorbed carbon dioxide transported from the low pressure absorber (29) to the high pressure absorber (33),
transporting a rich solvent stream (37) containing ammonia and a higher content of carbon dioxide emitted from the high pressure absorber (33) to a urea production plant (38) for the production of urea,
**characterised by** recycling stream (39) containing carbamate in a solution or slurry also containing ammonia, carbon dioxide, and
water from the urea production plant (38) to the low pressure absorber (29), and
transporting stream (43) from the ammonia production unit (41) to the urea production plant (38).

2. The method according to claim 1, wherein the flue gas stream (25) is obtained during combustion in a stream reformer (14) in the ammonia production process.

3. The method according to claims 1 and 2, wherein a syngas stream (26) containing hydrogen and carbon monoxide, and formed during a steam reforming reaction of the ammonia production process, is transported to a carbon monoxide shift unit (27) in which the carbon monoxide (26) is converted into carbon dioxide to form the hydrogen/carbon dioxide stream (28).

4. The method according to any one of claims 1-3, wherein a nitrogen containing stream (35) is emitted from the low pressure absorber (29), and a hydrogen stream (34) is emitted from the high pressure absorber (33), wherein both the nitrogen containing stream (35) and the hydrogen containing stream (34) are transported to the ammonia production unit (41) and are reacted for the production of ammonia.

5. The method according to any one of claims 1-4, wherein an ammonia stream (40), optionally originating from the ammonia production unit (41), is added to the recycle stream (39) used to generate a lean solvent stream (42) containing ammonia and a low content of carbon dioxide.

6. The method according to any one of claims 1-5, wherein a slip stream (36) diverted from the rich solvent stream (37) is transported the low pressure absorber (29).

7. The method according to any one of claims 1-6, wherein a partial stream (32) of the lean solvent stream (42) is added to the high pressure absorber (33) or to the semi rich solvent stream (30).

8. The method according to any one of claims 1-7, wherein a partial stream (31) of the semi rich solvent stream (30) is transported to the urea production plant (38).

9. The method according to any one of claims 1-8, wherein the pressure in the low pressure absorber (29) is 0.08-0.15 MPa, preferably 0.095-0.11 MPa, and the pressure in the high pressure absorber (33) is 0.3-4.0 MPa, preferably 0.095-0.11 MPa.

10. The method according to any one of claims 1-9, wherein the concentration of ammonia in the lean solvent stream (42), in the semi rich solvent stream (30), and in the rich solvent stream (37) is 2-12, preferably 6-9, moles per liter solution.

11. The method according to any one of claims 1-10, wherein the concentration of carbon dioxide in the lean solvent stream (42) is 0.2-4.0, preferably 1.5-4.0, moles per liter solution, the concentration of carbon dioxide in the semi rich solvent stream (30) is 1.0-5.5, preferably 2.5-4.5, moles per liter of solution, and the concentration of carbon dioxide in the rich solution stream (37) is 1.0-10.0, preferably 3.0-6.0, moles per liter solution.

12. The method according to any one of claims 1-11, wherein the ratio between the concentration of ammonia and the concentration of carbon dioxide in the streams (42), (30), and (37), respectively, is 1:1 to 3:1, preferably 3:2 to 5:2.

13. A system for the method according to any one of claims 1-12 of forming urea by integration of a part of an ammonia production process with that of a urea production process, wherein it comprises a steam reformer (14) or a gasification reactor of an ammonia production plant, a low pressure absorber (29), a high pressure absorber (33), and a urea production plant (38), wherein a conduit for a flue gas stream (25) is connected between the steam reformer (14), the gasification reactor, or another flue gas source, and the low pressure absorber (29), wherein a conduit for a hydrogen/carbon dioxide stream (28) is arranged between the steam reformer (14) or the gasification reactor and the high pressure absorber (33), wherein a conduit for a semi rich solvent stream (30) is fluidly connected between the low pressure absorber (29) and the high pressure absorber (33), wherein a conduit for a rich solvent stream (37) is fluidly connected between the high pressure absorber (33) and the urea production plant (38), and wherein a conduit for a lean solvent stream (42) is arranged between the urea production plant (38) and the low pressure absorber (29).

14. The system according to claim 13, wherein it also comprises an ammonia production unit (41), a conduit for a hydrogen stream (34) fluidly connected between the high pressure absorber (33) and the ammonia production unit (41), a conduit for a nitrogen containing stream (35) fluidly connected between the low pressure absorber (29) and the ammonia production unit (41), and a conduit for a stream (43) of the balance of ammonia fluidly connected between the ammonia production unit (41) and the urea production plant (38).

15. The system according to any one of claims 13 and 14, wherein it comprises a carbon monoxide shift unit (27) for the conversion of carbon monoxide into carbon dioxide, wherein a conduit for a syngas stream (26) containing hydrogen and carbon monoxide is fluidly connected between the steam reformer (14) or the gasification reactor and the carbon monoxide shift unit (27), and wherein the conduit for the hydrogen/ carbon dioxide stream (28) is fluidly connected between the carbon monoxide shift unit (27) and the high pressure absorber (33).

16. The system according to any one of claims 13-15, wherein it comprises a conduit for a partial stream (31) of the semi rich solvent stream (30) fluidly connected between the conduit for the semi rich solvent stream (30) and the conduit for the rich solvent stream (37).

17. The system according to any one of claims 13-16, wherein a conduit for a recycle stream (39) containing carbamate in a solution or in a slurry, also containing ammonia, carbon dioxide and water, is fluidly connected between the urea production plant (38) and the conduit for the lean solvent stream (42), and wherein a conduit for an ammonia stream (40) is connected to the conduit for the recycle stream (39).

18. The system according to any one of claims 13-17, wherein a conduit for a partial stream (32) of the lean solvent stream (42) is fluidly connected between the conduit for the lean solvent stream (42) and the conduit for the semi rich solvent stream (30) or the high pressure absorber (33).

## Patentansprüche

1. Verfahren zum Bilden von Harnstoff durch Integration eines Teils eines Ammoniakherstellungsprozesses in einen Harnstoffherstellungsprozess, umfassend:
Erhalten eines Wasserstoff/Kohlendioxidstroms (28) aus einem Ammoniakherstellungsprozess und eines Rauchgasstroms (25), der Kohlendioxid und Stickstoff enthält,
Transportieren des Rauchgasstroms (25) zu einem Niedrigdruckabsorber (29), der den Wasserstoff/Kohlendioxidstrom (28) zu einem Hochdruckabsorber (33) transportiert, wobei ein halbfetter Lösungsmittelstrom (30) verwendet wird, der absorbiertes Kohlendioxid enthält, das von dem Niedrigdruckabsorber (29) zu dem Hochdruckabsorber (33) transportiert wird,
Transportieren eines fetten Lösungsmittelstroms (37), der Ammoniak und einen höheren Gehalt von Kohlendioxid enthält und der von dem Hochdruckabsorber (33) an eine Harnstoffherstellungsanlage (38) zur Produktion von Harnstoff abgegeben wird,
**gekennzeichnet durch**
einen Rückgewinnungsstrom (39), der Carbamat in einer Lösung oder einer Schlacke enthält, die auch Ammoniak, Kohlendioxid und Wasser aus der Harnstoffherstellungsanlage (38) enthält, zu dem Niedrigdruckabsorber (29), und
einen Transportstrom (43) von der Ammoniakherstellungsanlage (41) zu der Harnstoffherstellungsanlage (38).

2. Verfahren nach Anspruch 1, wobei der Rauchgasstrom (25) während einer Verbrennung in einem Dampfreformer (14) in dem Ammoniakherstellungsprozess erhalten wird.

3. Verfahren nach Anspruch 1 und 2, wobei ein Synthesegasstrom (26), der Wasserstoff und Kohlenmonoxid enthält und während einer Dampfreformierungreaktion des Ammoniakherstellungsprozesses gebildet wird, zu einer Kohlenmonoxidübergangseinheit (27) transportiert wird, in der das Kohlenmonoxid (26) in Kohlendioxid umgewandelt wird, um den Wasserstoff/Kohlendioxidstrom (28) zu bilden.

4. Verfahren nach einem der Ansprüche 1 bis 3, wobei ein Stickstoff enthaltender Strom (35) von dem Niedrigdruckabsorber (29) abgegeben wird, und wobei ein Wasserstoffstrom (34) von dem Hochdruckabsorber (33) abgegeben wird, wobei sowohl der Stickstoff enthaltende Strom (35) als auch der Wasserstoffstrom (34) zu der Ammoniakherstellungseinheit (41) transportiert werden und für die Herstellung von Ammoniak zu einer Reaktion gebracht werden.

5. Verfahren nach einem der Ansprüche 1 bis 4, wobei ein Ammoniakstrom (40), der optional von der Ammoniakherstellungseinheit (41) stammt, zu dem Rückgewinnungsstrom (39) hinzugefügt wird, der verwendet wird, um einen mageren Lösungsmittelstrom (42) zu erzeugen, der Ammoniak und einen niedrigen Gehalt von Kohlendioxid enthält.

6. Verfahren nach einem der Ansprüche 1 bis 5, wobei ein Überbrückungsstrom (36), der aus dem fetten Lösungsmittelstrom (37) abgeleitet wird, zu dem Niedrigdruckabsorber (29) transportiert wird.

7. Verfahren nach einem der Ansprüche 1 bis 6, wobei ein Teilstrom (32) des mageren Lösungsmittelstroms (42) zu dem Hochdruckabsorber (33) oder dem halbfetten Lösungsmittelstrom (30) hinzugefügt wird.

8. Verfahren nach einem der Ansprüche 1 bis 7, wobei ein Teilstrom (31) des halbfetten Lösungsmittelstroms (30) zu der Harnstoffherstellungsanlage (38) transportiert wird.

9. Verfahren nach einem der Ansprüche 1 bis 8, wobei der Druck in dem Niedrigdruckabsorber (29) 0,08 bis 0,15 MPa, vorzugsweise 0,095 bis 0,11 MPa beträgt und der Druck in dem Hochdruckabsorber (33) 0,3 bis 4.0 MPa, vorzugsweise 0,95 bis 0,11 MPa beträgt.

10. Verfahren nach einem der Ansprüche 1 bis 9, wobei die Konzentration von Ammoniak in dem mageren Lösungsmittelstrom (42), in dem halbfetten Lösungsmittelstrom (30) und in dem fetten Lösungsmittelstrom (37) 2 bis 12, vorzugsweise 6 bis 9 Mol pro Liter des Lösungsmittels beträgt.

11. Verfahren nach einem der Ansprüche 1 bis 10, wobei die Konzentration von Kohlendioxid in dem mageren Lösungsmittelstrom (42) 0,2 bis 4,0, vorzugsweise 1,5 bis 4,0 Mol pro Liter des Lösungsmittels beträgt, wobei die Konzentration von Kohlendioxid in dem halbfetten Lösungsmittelstrom (30) 1,0 bis 5,5, vorzugsweise 2,5 bis 4,5 Mol pro Liter des Lösungsmittels beträgt, und wobei die Konzentration von Kohlendioxid in dem fetten Lösungsmittelstrom (37) 1,0 bis 10,0, vorzugsweise 3,0 bis 6,0 Mol pro Liter des Lösungsmittels beträgt.

12. Verfahren nach einem der Ansprüche 1 bis 11, wobei das Verhältnis zwischen der Konzentration von Ammoniak und der Konzentration von Kohlendioxid in den Strömen (42), (30) bzw. (37) 1:1 bis 3:1, vorzugsweise 3:2 bis 5:2 beträgt.

13. System für das Verfahren zum Bilden von Harnstoff durch Integration eines Teils eines Ammoniakherstellungsprozesses in einen Harnstoffherstellungsprozess nach einem der Ansprüche 1 bis 12, wobei es umfasst: einen Dampfreformer (14) oder einen Vergasungsreaktor einer Ammoniakherstellungsanlage, einen Niedrigdruckabsorber (29), einen Hochdruckabsorber (33) und eine Harnstoffherstellungsanlage (38), wobei eine Leitung für einen Rauchgasstrom (25) zwischen den Dampfreformer (14), den Vergasungsreaktor oder eine andere Rauchgasquelle und den Niedrigdruckabsorber (29) eingebunden ist, wobei eine Leitung für einen Wasserstoff/Kohlendioxidstrom (28) zwischen dem Dampfreformer (14) oder dem Vergasungsreaktor und dem Hochdruckabsorber (33) angeordnet ist, wobei eine Leitung für einen halbfetten Lösungsmittelstrom (30) in einer Strömungsverbindung zwischen den Niedrigdruckabsorber (29) und den Hochdruckabsorber (33) eingebunden ist, wobei eine Leitung für einen fetten Lösungsmittelstrom (37) in einer Strömungsverbindung zwischen den Hochdruckabsorber (33) und die Harnstoffherstellungsanlage (38) eingebunden ist, und wobei eine Leitung für einen mageren Lösungsmittelstrom (42) zwischen der Harnstoffherstellungsanlage (38) und dem Niedrigdruckabsorber (29) angeordnet ist.

14. System nach Anspruch 13, wobei es auch umfasst: eine Ammoniakherstellungseinheit (41), eine Leitung für einen Wasserstoffstrom (34), die in einer Strömungsverbindung zwischen den Hochdruckabsorber (33) und die Harnstoffherstellungseinheit (41) eingebunden ist, eine Leitung für einen Stickstoff enthaltenden Strom (35), die in einer Strömungsverbindung zwischen den Niedrigdruckabsorber (29) und die Harnstoffherstellungseinheit (41) eingebunden ist, und eine Leitung für einen Ammoniakausgleichsstrom (43), die in einer Strömungsverbindung zwischen die Ammoniakherstellungseinheit (41) und die Harnstoffherstellungsanlage (38) eingebunden ist.

15. System nach einem der Ansprüche 13 und 14, wobei es eine Kohlenmonoxidübergangseinheit (27) für die Umwandlung von Kohlenmonoxid in Kohlendioxid umfasst, wobei eine Leitung für einen Synthesegasstrom (26), der Wasserstoff und Kohlenmonoxid enthält, in einer Strömungsverbindung zwischen den Dampfreformer (14) oder den Vergasungsreaktor und die Kohlenmonoxidübergangseinheit (27) eingebunden ist, und wobei die Leitung für den Wasserstoff/Kohlendioxidstrom (28) in einer Strömungsverbindung zwischen die Kohlenmonoxidübergangseinheit (27) und den Hochdruckabsorber (33) eingebunden ist.

16. System nach einem der Ansprüche 13 bis 15, wobei es eine Leitung für einen Teilstrom (31) des halbfetten Lösungsmittelstroms (30) umfasst, die in einer Strömungsverbindung zwischen die Leitung für den halbfetten Lösungsmittelstrom (30) und die Leitung für den fetten Lösungsmittelstrom (37) eingebunden ist.

17. System nach einem der Ansprüche 13 bis 16, wobei eine Leitung für einen Rückgewinnungsstrom (39), der Carbamat in einer Lösung oder einer Schlacke enthält, die auch Ammoniak, Kohlendioxid und Wasser enthält, in einer Strömungsverbindung zwischen die Harnstoffherstellungsanlage (38) und die Leitung für den mageren Lösungsmittelstrom (42) eingebunden ist, und wobei eine Leitung für einen Ammoniakstrom (40) mit einer Leitung für den Rückgewinnungsstrom (39) verbunden ist.

18. System nach einem der Ansprüche 13 bis 17, wobei eine Leitung für einen Teilstrom (32) des mageren Lösungsmittelstroms (42) in einer Strömungsverbindung zwischen die Leitung für den mageren Lösungsmittelstrom (42) und die Leitung für den halbfetten Lösungsmittelstrom (30) oder den Hochdruckabsorber (33) eingebunden ist.

## Revendications

1. Procédé de formation d'urée par intégration d'une partie d'un procédé de production d'ammoniac dans un procédé de production d'urée, comprenant :
l'obtention d'un courant d'hydrogène/dioxyde de carbone (28) provenant d'un procédé de production d'ammoniac, et d'un courant de gaz de carneau (25) contenant du dioxyde de carbone et de l'azote,
le transport du courant de gaz de carneau (25) jusqu'à un absorbeur basse pression (29),
le transport du courant d'hydrogène/dioxyde de carbone (28) jusqu'à un absorbeur haute pression (33) avec un courant de solvant semi-riche (30) contenant du dioxyde de carbone absorbé transporté depuis l'absorbeur basse pression (29) jusqu'à l'absorbeur haute pression (33),
le transport d'un courant de solvant riche (37) contenant de l'ammoniac et une teneur supérieure en dioxyde de carbone émis depuis l'absorbeur haute pression (33) jusqu'à une installation de production d'urée (38) pour la production d'urée,
**caractérisé par** le recyclage d'un courant (39) contenant du carbamate dans une solution ou suspension contenant également de l'ammoniac, du dioxyde de carbone et de l'eau provenant de l'installation de production d'urée (38) dans l'absorbeur basse pression (29), et
le transport d'un courant (43) depuis l'unité de production d'ammoniac (41) jusqu'à l'installation de production d'urée (38).

2. Procédé selon la revendication 1, dans lequel le courant de gaz de carneau (25) est obtenu pendant une combustion dans un vaporeformeur (14) dans le procédé de production d'ammoniac.

3. Procédé selon les revendications 1 et 2, dans lequel un courant de gaz de synthèse (26) contenant de l'hydrogène et du monoxyde de carbone, et formé pendant une réaction de vaporeformage du procédé de production d'ammoniac, est transporté jusqu'à une unité de déplacement de monoxyde de carbone (27) dans laquelle le monoxyde de carbone (26) est transformé en dioxyde de carbone pour former le courant d'hydrogène/dioxyde de carbone (28).

4. Procédé selon l'une quelconque des revendications 1 à 3, dans lequel un courant contenant de l'azote (35) est émis depuis l'absorbeur basse pression (29), et un courant d'hydrogène (34) est émis depuis l'absorbeur haute pression (33), le courant contenant de l'azote (35) et le courant contenant de l'hydrogène (34) étant tous deux transportés jusqu'à l'unité de production d'ammoniac (41) et étant mis à réagir pour la production d'ammoniac.

5. Procédé selon l'une quelconque des revendications 1 à 4, dans lequel un courant d'ammoniac (40), provenant éventuellement de l'unité de production d'ammoniac (41), est ajouté au courant de recyclage (39) utilisé pour générer un courant de solvant pauvre (42) contenant de l'ammoniac et une faible teneur en dioxyde de carbone.

6. Procédé selon l'une quelconque des revendications 1 à 5, dans lequel un courant de glissement (36) détourné depuis le courant de solvant riche (37) est transporté jusqu'à l'absorbeur basse pression (29).

7. Procédé selon l'une quelconque des revendications 1 à 6, dans lequel un courant partiel (32) du courant de solvant pauvre (42) est ajouté à l'absorbeur haute pression (33) ou au courant de solvant semi-riche (30).

8. Procédé selon l'une quelconque des revendications 1 à 7, dans lequel un courant partiel (31) du courant de solvant semi-riche (30) est transporté jusqu'à l'installation de production d'urée (38).

9. Procédé selon l'une quelconque des revendications 1 à 8, dans lequel la pression dans l'absorbeur basse pression (29) est de 0,08-0,15 MPa, de préférence 0,095-0,11 MPa, et la pression dans l'absorbeur haute pression (33) est de 0,3-4,0 MPa, de préférence 0,095-0,11 MPa.

10. Procédé selon l'une quelconque des revendications 1 à 9, dans lequel la concentration d'ammoniac dans le courant de solvant pauvre (42), dans le courant de solvant semi-riche (30) et dans le courant de solvant riche (37) est de 2-12, de préférence 6-9 moles par litre de solution.

11. Procédé selon l'une quelconque des revendications 1 à 10, dans lequel la concentration de dioxyde de carbone dans le courant de solvant pauvre (42) est de 0,2-4,0, de préférence 1,5-4,0 moles par litre de solution, la concentration de dioxyde de carbone dans le courant de solvant semi-riche (30) est de 1,0-5,5, de préférence 2,5-4,5 moles par litre de solution, et la concentration de dioxyde de carbone dans le courant de solvant riche (37) est de 1,0-10,0, de préférence 3,0-6,0 moles par litre de solution.

12. Procédé selon l'une quelconque des revendications 1 à 11, dans lequel le rapport entre la concentration d'ammoniac et la concentration de dioxyde de carbone dans les courants (42), (30) et (37), respectivement, est de 1:1 à 3:1, de préférence 3:2 à 5:2.

13. Système pour le procédé selon l'une quelconque des revendications 1-12 de formation d'urée par intégration d'une partie d'un procédé de production d'ammoniac avec celle d'un procédé de production d'urée, qui comprend un vaporeformeur (14) ou un réacteur de gazéification d'une installation de production d'ammoniac, un absorbeur basse pression (29), un absorbeur haute pression (33) et une installation de production d'urée (38), une conduite pour un courant de gaz de carneau (25) étant raccordée entre le vaporeformeur (14), le réacteur de gazéification ou une autre source de gaz de carneau, et l'absorbeur basse pression (29), une conduite pour un courant d'hydrogène/dioxyde de carbone (28) étant disposée entre le vaporeformeur (14) ou le réacteur de gazéification et l'absorbeur haute pression (33), une conduite pour un courant de solvant semi-riche (30) étant raccordée fluidiquement entre l'absorbeur basse pression (29) et l'absorbeur haute pression (33), une conduite pour un courant de solvant riche (37) étant raccordée fluidiquement entre l'absorbeur haute pression (33) et l'installation de production d'urée (38), et une conduite pour un courant de solvant pauvre (42) étant disposée entre l'installation de production d'urée (38) et l'absorbeur basse pression (29).

14. Système selon la revendication 13, qui comprend également une unité de production d'ammoniac (41), une conduite pour un courant d'hydrogène (34) raccordée fluidiquement entre l'absorbeur haute pression (33) et l'unité de production d'ammoniac (41), une conduite pour un courant contenant de l'azote (35) raccordée fluidiquement entre l'absorbeur basse pression (29) et l'unité de production d'ammoniac (41), et une conduite pour un courant (43) du reste d'ammoniac raccordée fluidiquement entre l'unité de production d'ammoniac (41) et l'installation de production d'urée (38).

15. Système selon l'une quelconque des revendications 13 et 14, qui comprend une unité de déplacement de monoxyde de carbone (27) pour la transformation de monoxyde de carbone en dioxyde de carbone, une conduite pour un courant de gaz de synthèse (26) contenant de l'hydrogène et du monoxyde de carbone étant raccordée fluidiquement entre le vaporeformeur (14) ou le réacteur de gazéification et l'unité de déplacement de monoxyde de carbone (27), et la conduite pour le courant d'hydrogène/dioxyde de carbone (28) étant raccordée fluidiquement entre l'unité de déplacement de monoxyde de carbone (27) et l'absorbeur haute pression (33).

16. Système selon l'une quelconque des revendications 13 à 15, qui comprend une conduite pour un courant partiel (31) du courant de solvant semi-riche (30) raccordée fluidiquement entre la conduite pour le courant de solvant semi-riche (30) et la conduite pour le courant de solvant riche (37).

17. Système selon l'une quelconque des revendications 13 à 16, dans lequel une conduite pour un courant de recyclage (39) contenant du carbamate dans une solution ou dans une suspension contenant également de l'ammoniac, du dioxyde de carbone et de l'eau, est raccordée fluidiquement entre l'installation de production d'urée (38) et la conduite pour le courant de solvant pauvre (42), et dans lequel une conduite pour un courant d'ammoniac (40) est raccordée à la conduite pour le courant de recyclage (30).

18. Système selon l'une quelconque des revendications 13 à 17, dans lequel une conduite pour un courant partiel (32) du courant de solvant pauvre (42) est raccordée fluidiquement entre la conduite pour le courant de solvant pauvre (42) et la conduite pour le courant de solvant semi-riche (30) ou l'absorbeur haute pression (33).
